# EUROPEAN PATENT SPECIFICATION

(11) **EP 3 284 396 B1**
(45) Date of publication and mention of the grant of the patent: **12.02.2020**
(21) Application number: 16184311.5
(22) Date of filing: 16.08.2016
(51) Int. Cl.: A61B 5/00, G01N 21/31

(54) **OBSERVATION APPARATUS AND METHOD FOR VISUAL ENHANCEMENT OF AN OBSERVED OBJECT**
BEOBACHTUNGSVORRICHTUNG UND VERFAHREN ZUR VISUELLEN VERBESSERUNG EINES BEOBACHTETEN OBJEKTS
APPAREIL ET PROCÉDÉ D'OBSERVATION POUR UNE AMÉLIORATION VISUELLE D'UN OBJET OBSERVÉ

(43) Date of publication of application: 21.02.2018
(73) Proprietor: Leica Instruments (Singapore) Pte. Ltd., 608924 Singapore (SG)
(72) Inventor: Themelis, George, 88131 Lindau (DE)
(74) Representative: Grünecker Patent- und Rechtsanwälte PartG mbB

(56) References cited:
- WO-A1-2016/127173
- US-A1- 2010 054 545
- US-A1- 2010 069 758
- US-A1- 2014 112 559
- US-A1- 2015 381 908

## Description

The invention relates to an observation apparatus and method for visual enhancement of an observed object, in particular a medical observation apparatus and method.

Imaging devices are known which utilize projectors to enhance visually the veins on the body of a patient by projecting an image directly on to the patient. These devices are used to increase visibility of the veins for clinical assistance, such as facilitating the setting of a syringe. The operating principle is based on an NIR camera which images the tissue using an NIR spectrum in which veins exhibit a higher contrast to the surrounding tissue than in the visible-light range. The visible-light range designates electromagnetic radiation with wavelengths from about 390 to 700 nm. The contrast of the NIR image is enhanced and displayed as an image in the visible-light range on the skin by means of the projector. The veins of the image are projected onto the veins of the body which are thus made more visible.

In document US 2010/069758 A1, systems and methods for hyperspectral medical imaging using real-time projection of spectral information are disclosed. Upon illumination of an object, each region is resolved into a corresponding spectrum. An image based on a selected portion of each spectrum may be constructed and projected onto the subject.

In US 2015/381908 A1 a system for hyperspectral imaging in visible light, a method for recording a hyperspectral image and a display device for displaying the recorded hyperspectral image in visible light is disclosed. The hyperspectral imaging system comprises a light source a projector and a camera, which share a common coaxial optical axis, such that a projection of the projector is aligned to the associated tissue of the object.

This visualization method allows easy and direct visual inspection of the patient but suffers from several drawbacks.

First, this technology relies on using an NIR camera which does not have sensitivity in the visible-light range of the image projected onto the patient. Thus, the camera input is not influenced by the projection of visible-light images onto the area which is observed by the NIR camera. The technology cannot be expanded to enhance visibility of features that need to be captured in the visible-light range.

Another drawback is that the projected image, which is based on an image in the non-visible-light range, does not necessarily reflect the actual visual appearance of the vein. The extent and boundaries of the veins in the non-visible light range may not coincide with the extent and boundaries in the visible-light range.

Finally, the contrast of the image projected onto the patient depends on the ambient light conditions. In very bright surroundings, the brightness of the projector may not be sufficient to produce high contrasts on the patient. Further, visibility of the projected image depends on the reflectance of the surface onto which the image is projected. For example, wet surfaces, such as bloody surfaces or surfaces treated with an ointment may obscure the projected image.

It is the aim of the invention to provide an observation apparatus and method for visual enhancement of an observed object which does not have the above-mentioned drawbacks and thus can be used for a wider range of imaging applications, such as for example direct visualization of tumors, lymph nodes, and that can also be used in combination with fluorescent tissue markers.

According to the invention, this aim is achieved by the observation apparatus according to claim 1.

Further, the above aim is achieved by the method of claim 8.

The apparatus and method according to the invention allows to visualize features of the object right on the object where these features are observed. The input multispectral camera records the input color images using a first set of at least two spectral bands. Thus, the input images correspond to what an observer would see by looking at the object. The output color image projector projects onto the projection zone output color images using a different set of spectral bands, but also in the visible-light range. The projection of the output color images does not influence the capturing of the input color images, as both are composed of different spectral bands, which do not interfere. Although the observer sees a superposition of the output color image and the reflections from the ambient light on the projection area, the input camera system does not capture the reflections from the output color images.

By modifying colors, contrast or brightness in the output color image, and/or restricting these changes to certain patterns recognized by the apparatus in the input color image, specific features of the object can be visually enhanced right on the object to increase visibility for an observer or facilitate recognition of certain features. Further, the quality of the output color images can be improved by performing noise reduction or spatial deconvolution algorithms.

If the input camera system is also sensitive to input spectral bands in the NIR or IR-range, the output color images may be used to represent IR- or NIR-image data in the visible-light range. This allows e.g. to display areas of different temperature and/or areas, which are marked by a fluorescent tissue marker, in the visible-light range directly on the object without impairing the capturing of the input color images.

The invention can be improved by the following features which can be combined independent of one another.

According to one aspect, the output spectral bands have a different color appearance. The output spectral bands may comprisepredetermined narrow-band spectral bands such as e.g. generated by lasers of different colors. In particular, the output spectral bands may be narrower and/or less in number than the output spectral bands. Thus, more and/or wider spectral bands are available for the camera system, which allows to capture a greater variety of features of the object, which may be present in only one spectral band.

In order to be able to display all colors using the output spectral bands, it is preferred that the output spectral bands form a preferably additive color space. In particular, a tri-band output spectrum, such as an RGB spectrum can be used. This approach only excludes three spectral bands from the input spectrum and thus allows most information gathered from the object in the input image for being captured by the camera system.

The input color image and output color image should be spatially congruent and of the same orientation in the projection area. This makes sure that features in the projected output color image are congruent and aligned with, or mapped identically onto, the corresponding features of the object.

It is further preferred that the projection area of the output color image projector is identical to a field of view or observation area of the camera system. Thus, the output color images cover all that is seen by the camera system.

Preferably, there is no overlap between the input spectral bands and the output spectral bands. This makes sure that the output color images cannot be captured by the camera system.

If a projector and/or a camera is used which is not limited to specific spectral bands, spectral separation of the input color images from the output color images can be achieved if the output color image projector comprises a band-pass output filter system which blocks the input spectral bands and/or in that the camera system comprises a band-pass input filter system which blocks the output spectral bands.

If the projector uses narrow-band and discrete output spectral bands, as in particular generated by one or more lasers of different colors, simple notch filters may be used to block the output spectral bands. A projector may e.g. use at least one of a blue, red and green laser to project color images onto the object.

In the context of this description, the expression *"blocking spectral bands"* comprises both eliminating and attenuating the spectral bands. However, elimination is preferred.

The observation apparatus may further comprise an image processor which is connected to the camera system for receiving the input color images and connected to the output color image projector for outputting the output color images. The input color images may contain input pixels and the output color images may contain output pixels. The image processor may be a hardware device which is dedicated to a specific task, e.g. image processing, by its hardware layout. The image processor may additionally or alternatively comprise a general-purpose computer which is configured to execute image processing software.

The image processor may be configured to compute the output color images automatically based on the input color images. This computation may include conversion of the data formats of the input color image and the output color image such as transforming the images between different color spaces and/or color representations and/or resolutions both with regard to spatial resolution and/or color resolution. The computation of the output color images may further be dependent on the reflective distribution across the projection area to compensate differences in reflectance across the projection area.

Further, the image processor may be configured to perform automatic pattern recognition and/or automatic image enhancement. For example, at least one area of the output color image may have at least one of a modified color, brightness and contrast compared to the at least one spatially corresponding area in the input image. For color modification, a color in the input color image may be replaced by a pseudo-color, in particular a neon color, which does not occur in nature. The image processor is preferably configured to compute this color modification.

The processing of the image processor is, according to one aspect of the invention, done in real-time. Thus, an output color image may be projected onto the projection area based on an input color image before the next input color image in the time sequence is captured by the camera system.

The time sequence of output color images is projected preferably at a frame rate which is higher than the flicker fusion rate, to reduce tiring effects for the observer and to provide smooth transition between subsequent input color images. The capture rate in which the camera system captures subsequent input color images is preferably larger than the flicker fusion rate. The frame rate of the output color images may be lower than the capture rate of the input color images. In particular, an output color image may be based on a number of subsequent input images. For example, subpixel-shifting of subsequent input images may be used to increase spatial resolution in a single output color image. Basing the output color image on a number of input color images may also be used to compute output color images having an increased dynamic range, such as HDR images.

According to a further aspect of the invention, the viewer may be an ocular or an observation screen. The viewing band-pass filter system only lets pass the output spectral bands which constitute the output color image. Reflections at other spectral bands are blocked and thus contrast is increased while at the same time allowing to view the object and the output color images at the output spectral bands.

The input spectral bands in which the multispectral camera is sensitive may include at least one of the primary colors, such as red, green and blue. The input spectral bands may be overlapping or discrete. The output spectral bands may be overlapping or discrete. Discrete output spectral bands may e.g. be generated by lasers of different color.

Preferably, at least one of the at least two spectral bands of the multispectral camera system is in the visible light range. The multispectral camera system may in particular be a RGB camera. According to another advantageous aspect, the camera system may be a multispectral camera having an input spectrum which consists of more than four input spectral bands. In particular, the camera system may comprise at least one imaging spectrometer and/or a hyperspectral camera. This camera allows to simultaneously capture image data in a large number of spectral bands. An imaging spectrometer does not necessarily need an input filter system for blocking the output spectral bands, as instead, only the image data at the input spectral bands may be read out of the camera system while data at the input spectral bands are dismissed. Alternatively, the input spectrum may be selected to not overlap with the output spectrum.

To further reduce reflections from the object, at least one of the output color image projector and the viewer may comprise a polarization filter system. For example, the output color image projector may comprise a linear polarizing filter and the viewer may comprise an adjustable polarization filter system comprising e.g. one rotatable linear polarizing filter. This allows to adjust the attenuation of intensity and of reflections according to the observer's needs.

According to another aspect of the disclosure, an illumination system may be comprised. Although a simple illumination system such as a light source with a continuous illumination spectrum which may include spectral bands which stimulate fluorescence in tissue-marking fluorophores, may be used, it is preferred that the illumination system comprises an illumination color image projector. Thus, instead of merely providing a diffuse illumination by a light source such as an LED or light bulb assembly, illumination is performed by projecting an illumination color image onto the object. Consequently, the illumination of the projection area or of the observation area of the camera system can be controlled to the accuracy of a pixel in the projected illumination color image.

In particular, the illumination color image projector may be provided for projecting a time sequence of illumination color images onto the projection area. Preferably, the observation apparatus, in particular its image processor, is configured to compute the illumination color images based on the input color images, in particular in real time, and project them, in particular in real time, onto the projection area. Computation and projection of the illumination color images based on input color images is preferably performed by the image processor before the next input color image is captured.

Using an illumination color image for illumination allows to exactly match the illumination to the spatial distribution of at least one of the reflectance, absorption, fluorescence and transmission of light across the projection or observation area. The observation apparatus, in particular its image processor is preferably adapted to compute a distribution of the reflectance of the object across the projection area. This distribution may then be used for computation of the output color images to obtain optimum contrast. In particular, the distribution which is obtained for the illumination spectral bands may simply be interpolated by the image processor to cover the output spectral bands between the illumination spectral bands.

In particular, the illumination color image projector may be controlled to adapt locally the intensity of the illumination in spectral bands, in which fluorescence is excited, to the intensity of the fluorescence. Areas with a high fluorescence intensity may be illuminated in the excitation spectral bands with a smaller intensity than areas, in which the fluorescence intensity is lower.

In order to avoid interference between the illumination color image and the output color image, the illumination color image projector has preferably an illumination spectrum in which the output spectral bands are at least partly blocked. For this, an illumination filter system can be used which comprises band-stop filters for the output spectral bands. In particular, the illumination spectrum may correspond to the input spectrum. If, however, the input spectrum contains fluorescence spectral bands, in which e.g. fluorophores emit fluorescence, as input spectral bands, it is preferred that the illumination spectrum does not contain the fluorescence bands. This avoids decreasing the contrast in the input color images of the fluorescence bands.

To avoid deterioration of the contrast of the output color images, in particular if the output color images are observed through a viewer as described above, the illumination spectrum does not overlap with the output spectrum at least in the visible-light range.

The illumination color image compensates the light-reflecting, absorbing, transmitting and/or fluorescence characteristics of the object surface across the observation area. It allows to lighten-up specific areas, which would otherwise be too dark for a full color resolution of the camera system.

In order to make full use of the dynamic capabilities of the camera system, the illumination color image may be at least in sections, preferably as a whole, a negative color image of the input color image. The observation apparatus, in particular its image processor, may be configured to compute the illumination color image as the negative color image of the input color image in real-time.

An observation apparatus comprising an illumination system in one of the above embodiments, a camera system in one of the above embodiments, an output color image projector in one of the above embodiments, and/or a viewer in one of the above embodiments in general uses two distinct imaging systems which operate on the same area, i.e. the observation or projector area on the object, and are functionally separated from each other by using the different input and output spectral bands. The output color image is used for viewing and represents the result of the image enhancement process. The camera system and the illumination system are used to optimize the input color image, which forms the basis of the image enhancement process and the output color images. The functional separation allows to optimize the input image captured independently of the result projection, although both use the same projection area.

It is preferred that the input color images and the output color images and preferably also the illumination color images are spatially congruent and have the same orientation. Congruence and orientation of these images ensures that the output images give an exact rendition of what is observed by the camera system and what is present on the object.

The observation apparatus, in particular its image processor, is preferably configured to transform and rectify the input color images, illumination color images and/or output color images preferably using the input color images as the base for adjusting the output and/or illumination color images.

According to another aspect of the disclosure, a common optical system is provided for the output color image projector and the camera system. The common optical system may comprise a common lens system. The common lens system ensures alignment of the optical axes of the output color image projector and the camera system. It also ensures congruence of the observation area of the camera system and the projection area onto which the illumination system and the output color image projector project their images. The common optical system may also comprise fibre optics if at least one of the camera system, output color image projector and viewer is located remote from the object.

A beam splitter arrangement may be used to separate the light from the illumination system and/or the output color image projector into the optical system from the light from the projection or observation area.

The method according to the disclosure can be further improved by comprising the step of illuminating the observation area by projecting thereon an illumination color image, and basing the illumination color image on the input color image.

The method may further comprise the step of illuminating the observation area with an illumination spectrum consisting of the input spectral bands.

The method may also comprise the step of adapting the illumination color images to compensate differences in at least one of light reflectance, fluorescence, transmission and absorption across the observation area, at least in the visible-light range.

The method may further comprise the step of computing the illuminating color images as the color negative of the input color images.

The step of computing the output color images based on the input color images may comprise the step of replacing a color in the input color image in at least one coherent area by another color, preferably a pseudo-color, in the output color image.

The step of computing the output color image based on the input color images may comprise the steps of changing at least one of color, intensity and contrast in the output color images compared to the input color images.

In the following, the invention is described in detail with reference to the drawings using an exemplarily embodiment.

In the figures, elements, which correspond to each other with respect to function and/or structure are marked with the same reference numeral.

Further, it is to be understood that the combination of features which is exemplarily shown in the embodiment of the accompanying drawings, one or more features can be omitted if their technical effect as described above is not needed for a specific application. Conversely, one or more figures may be added to the embodiment if their technical effect as described above is beneficial for a specific application of the invention.

In the figures:
- Figure 1: shows a schematic representation of an observation apparatus according to the invention;
- Figure 2: shows a schematic representation of a usage of the observation apparatus according to the invention;
- Figure 3: shows a schematic representation of the observation apparatus shown in Figure 2.

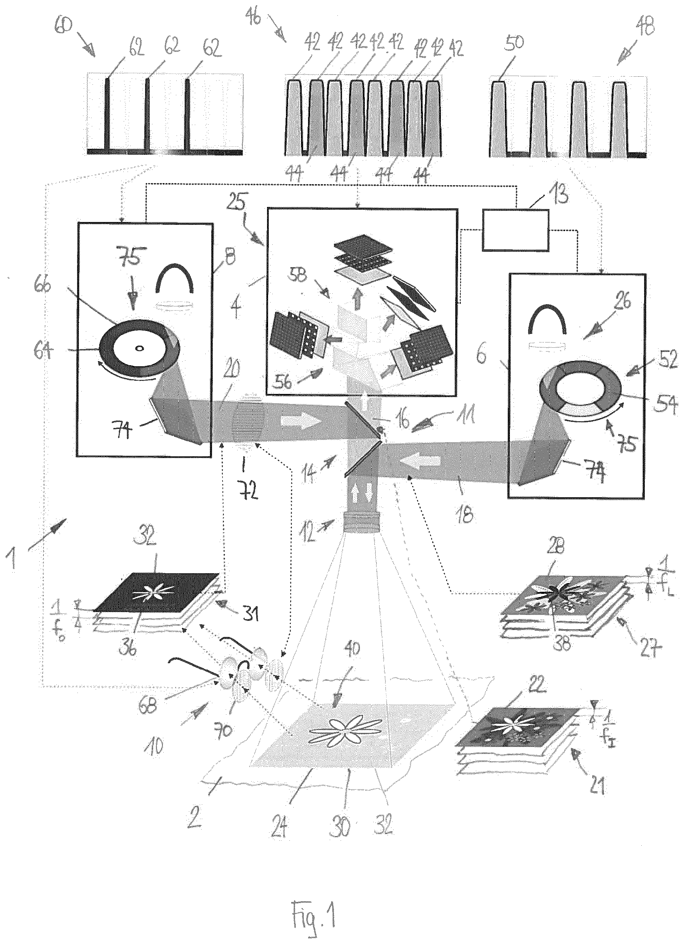

First, the structure and function of an observation apparatus 1 for visual enhancement of an observed object 2 is described with reference to Figure 1. The observed object 2 may in particular be image tissue such as the body of a human, an animal and/or a plant.

The observation apparatus 1 comprises a camera system 4, an illumination system 6, an output color image projector 8, a viewer 10, a lens system 12, an image processor 13 and a beam splitter system 14.

The light path 16 of the camera system 4, the light path 18 of the illumination system 6 and the light path 20 of the output color image projector 8 are all directed via the beam splitter system 14 through an optical system 11 onto the object 2. The optical system 11 may comprise a lens system 11 for aligning the light paths 16, 18, 20.

The camera system 4 is configured to capture a time sequence 21 of color input images 22 from an observation area 24 on the object 2 at a frame rate f_{I}. The camera system may comprise at least one imaging spectroscope 25 such as a spectral camera or hyperspectral camera.

The illumination system 6 may comprise an illumination color image projector 26 which projects a time sequence 27 of illumination color images 28 on a projection area 30 on the object 2 at a frame rate f_{L}. The projection area 30 is preferably spatially congruent to the observation area 24. Alternatively a conventional illumination device (not shown) or ambient illumination may be used.

The illumination color images 28 in the time sequence 21 are computed by the observation apparatus 1, in particular its image processor 13, based on the input color image 22.

The illumination color images 28 in the time sequence 27 are preferably congruent and of the same orientation as the corresponding color input images 22 in the time sequence 21 captured by the camera system 4 on the observation area 24.

The output color image projector 8 projects a time sequence 31 of output color images 32 at a frame rate fₒ onto a projection area 34 of the output color image projector 8. The frame rate fₒ may be independent of the frame rate fₗ of the camera system 4. The projection area 34 is preferably spatially congruent with at least one of the projection area 30 and the observation area 24. The output color images 32 have, in the projection area 34, features 36 that are spatially congruent and of the same orientation as corresponding features in the illumination color image 28 and/or the color input image 22 and/or on the object 2 for each image in the time sequence. Thus, the images 28 and 32 are congruent to each other on the object 2 and with the object 2.

The output color images 32 are computed by the observation apparatus 1, in particular its image processor 13, based on the input color images 22. The viewer 10 may be provided to an observer for viewing the common projection area 40 which corresponds to the projection areas 34, 30 and the observation area 24.

The camera system 4 and the illumination system 6 on the one hand and the output color image projector 8 and the viewer 10 on the other form two separate imaging subassemblies of the observation apparatus 1. Both subassemblies work on the same part of the object 2, namely the projection area 40 but are decoupled from each other by using different spectra operating at preferably non-overlapping spectral bands and being linked solely through the image processing performed by the observation apparatus 1 or its image processor 13, respectively. This separation results in a visual enhancement of arbitrarily selectable features of the object 2 without impairing image capture by the camera system 4. The subassembly comprising the camera system 4 and the illumination system 6 is used for providing color input images 22 with optimum quality. The subassembly comprising the output color image projector 8 and the viewer 10 is for viewing processed color input images 22 in the form of the color output images 32 directly on the object 2. This is explained in the following.

The camera system 4 is preferably a multispectral camera system that performs imaging at multiple, i.e. at least two, preferably more than four input spectral bands 42. Prefarably at least one input spectral band 42 is located in the visible-light range. At least one input spectral band may also be located in the NIR and/or IR range. The input spectral bands 42 may comprise fluorescent spectral bands 44 which correspond to fluorescence emission spectral bands of fluorescent materials on or in the object 2, such as fluorescent tissue markers or other fluorophores.

The combination of input spectral bands 42 defines the input spectrum 46 of the camera system 4.

The illumination system 6 has an illumination spectrum 48 consisting of preferably non-overlapping illumination spectral bands 50. The illumination spectrum 48 corresponds to the input spectrum 46 preferably excluding any fluorescence spectral bands 44 if such are present. Thus, the illumination spectral bands 50 correspond to the input spectral bands 42 in case there is no fluorescence emission from the object in the respective spectral band.

The illumination color images 28 are superposed onto the object 2 in the projection area 40 and reflected by the object 2 depending on the distribution of reflectance across the projection area 40. Thus, the input color images 22 are directly influenced by the illumination color images 28 projected onto the projection area 40. In addition, any fluorescence triggered by the illumination spectrum 50 is also captured by the camera system 4.

Preferably, the illumination color images 28 are computed to be the color negatives of the respective input color images 22 for each of the images in a time sequence 21, 27. This maximizes the utilization of the dynamic range of the camera system 4. The computation is performed by the observation apparatus 1, in particular its image processor 13, in real time.

The illumination color images 28 may be computed to compensate other effects. For example, the intensity of illumination in a fluorescence excitation spectral band may be increased in an area, in which the fluorescence excited by this particular spectral band is low and vice versa.

Thus, a new illumination color image 28 is computed and projected onto the projection area 40 preferably within the time 1/fₗ between successive input color images 22 in a time sequence 21.

The illumination color image projector 26 may include an illumination filter system 52 which comprises band-pass or band-stop illumination filters 54 which block, i.e. attenuate or preferably eliminate, light outside the illumination spectral bands 50.

The camera system 4 may comprise an input filter system 56 which may be part of an internal beam splitter 58 which may direct light to different cameras which together form the camera system 4. If an imaging spectroscope is used, an input filter system 56 may not be necessary. In this case, input color images are put together simply by discarding any image data outside a input spectral band 42 from the imaging spectroscope.

The output color image projector 8 emits an output spectrum 60 consisting of output spectral bands 62. The output spectral bands 62 are separate from and do not overlap with the input spectral bands 42 and the illumination spectral bands 50. For a human observer it is sufficient for the output spectrum 60 to contain only three output spectral bands 62 which together form a color space such as an RGB space and which may overlap. The output color image projector 8 may comprise an output filter system 64 which restricts the output spectrum 60 to the output spectral bands 62. The output filter system 64 may towards this end comprise band-pass or band-stop output filters 66 which block in particular the input spectral bands 42.

Alternatively, the output color image projector 8 may be a laser projector using at least one laser, preferably a set of lasers of different color, which may form an additive color system. Using color lasers has the advantage that the spectral bands in the different colors do not overlap and may be blocked easily by using a notch filter.

As the output spectral bands 62 do not overlap the input spectral bands 42, the camera system 4 cannot detect the output color images 32 even though they are projected onto the common projection area 40. Moreover, the output color images 32 projected onto the projection area 40 are not influenced by the illumination color images 28 as they do not share common spectral bands. The output spectral bands 62 are preferably narrower than the input spectral bands 42. The number of output spectral bands 62 is preferably larger than the number of input spectral bands 42.

The viewer 10 comprises a viewer filter system 68 which is designed to block the illumination spectral bands 50 and preferably only lets pass the output spectral bands 62. The viewer filter system 68 may correspond to the output filter system 64 in its band-pass or band-stop characteristics. Thus, an observer looking through the viewer 10 only observes the output images 32 but does not see the illumination color images 28 as they are built using spectral bands 50 which are blocked by the viewing filter system 68. This greatly enhances the contrast of the output color images 32.

The image processor 13 may perform any image processing on the input color images 22 to generate the output color images 32, such as noise reduction, spatial deconvolution to compensate the light scattering effects of the object, replacing a color in the input color images 22 by a pseudo-color or any naturally occurring color, adding marks such as symbols and letters, and/or enhancing contrast and/or color and/or brightness distributions. Furthermore, the color output images 32 may be computed from captured fluorescence images, e.g. by displaying a ratio of the fluorescence intensities in false colors. The output color images 32 containing any of these modifications of the input color images 22 are projected directly onto the object 2 at exactly the position where the visually modified features are present on the object 2.

Using the multispectral illumination color image projector 26 and the multispectral camera system 4 allows to capture images of optimum quality as the illumination may be adjusted on a pixel basis to the reflectance and fluorescence characteristics of the object 2 and the characteristics of the camera system 4. A real time computational comparison of the illumination color images 32 and the input color images, e.g. done by the image processor 13 yields the distribution of reflectance across the projection area 40 in the output spectrum 48. This distribution may be interpolated by the image processor in real time to also cover the spectral bands 62 of the output spectrum 60 and for compensation of the output color images 32.

Reflections in the output spectral bands 62 may be reduced by providing a polarization filter system 70 with the viewer 10 which may be combined with an optional polarization filter 72 in the light path 20. The polarization filter 72 may be part of the output color image projector 8. For example, linear polarization may be used in the polarization filter system 70 and the polarization filter 72 and the polarization filter system 70 may be adjustable by e.g. providing a rotatable linear polarization filter.

The observation apparatus 1 as shown in Figure 1 may be simplified. For example, it may be sufficient to use an illumination system 6 providing a continuous illumination spectrum 48 instead of illumination spectral bands 50, or to use ambient light for illumination of the object. Such an illumination may result in a comparatively lower quality of the input color images 22. It is, however, still possible to compute output color images 32 based on the input color images 22 and to project them onto the projection area 40 without the output color images 32 being captured by the camera system 4 as long as the input spectral bands 42 and the output spectral bands 62 do not overlap.

The viewer 10 does not need to be part of the observation apparatus 1. It may be sufficient to project the output color image 32 onto the projection zone 40 so that an observer is able to view the superposition of the output color images 32 and the illumination color images 28 on the projection zone 40.

The output spectrum 60 contains preferably output spectral bands 62 exclusively in the visible-light range.

The illumination color image projector 52 and the output color image projector 8 are shown to comprise each a digital light processor 74 and a rotating filter wheel 75. Of course, any other type of color image projector may be used for any of the projectors 8, 26.

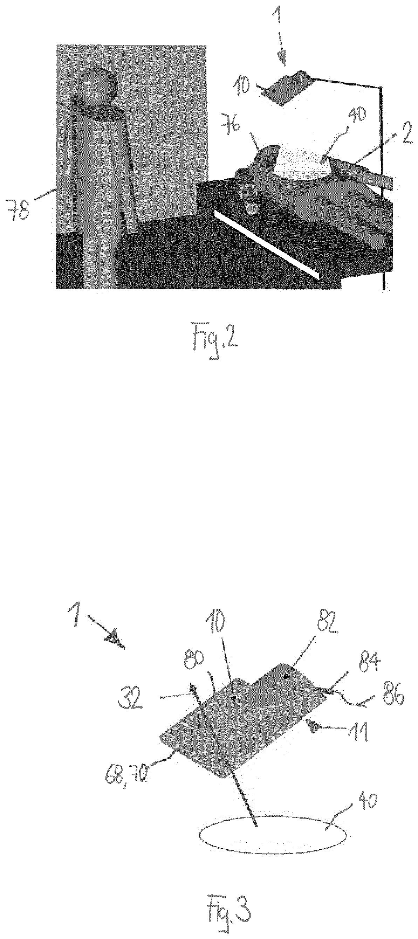

Figure 2 shows an example of an observation apparatus 1 which is used for observing an observation area 40 on a patient 76 as the object 2. The observation area 40 can be on the skin of the patient 76 or be a part of the patient 76 undergoing surgery. An observer 78, such as a surgeon or a medical assistant, may inspect the observation area 40 through the viewer 10 which in this case is a transparent screen 80 which preferably incorporates the viewer filter system 68 and optionally also a polarization filter system 70.

The observation apparatus of Figure 2 again shown in Figure 3, where the viewing screen 80 in which the viewer filter system 68 and optionally the polarization filter system 70 are incorporated. The viewer is held by a housing 82 which is supported by an adjustable stand 84. In the housing 82, at least the optical system 11 is received. The housing 82 may further also contain at least one of the camera system 4, and the output color image projector 8.

Alternatively, these devices may be arranged remote from the viewer 10 and fiber optics 86 as part of the optical system 11 may be employed to carry the light to and from the projection zone 40. The fiber optics may be received in the stand 84.

As described with reference to Figure 1, the observation apparatus 1 may also comprise an illumination system 6 as shown above, which is either received in the housing 82 or connected via the fiber optics 86.

### Reference Numeral List

- 1.: Observation apparatus
- 2.: Object
- 4.: Camera system
- 6.: Illumination system
- 8.: Output color image projector
- 10.: Viewer
- 11.: Optical system
- 12.: Lens system
- 13.: Image processor
- 14.: Beam splitter system
- 16: Light path of the camera system
- 18.: Light path of the illumination system
- 20.: Light path of the outward color image projector
- 21.: Time sequence of color input images
- 22.: Color input image
- 24.: Observation area of camera system
- 25.: Imaging spectroscope
- 26.: Illumination color image projector
- 27.: Time sequence of illumination color images
- 28.: Illumination color image
- 30.: Projection area of illumination color image projector
- 31.: Time sequence of output color images
- 32.: Output color images
- 34.: Projection area of output color image projector
- 36.: Feature in output color images
- 38.: Feature of illumination color image
- 40.: Common projection and observation area
- 42.: Input spectral bands
- 44.: Fluorescence spectral bands
- 46.: Input spectrum
- 48.: Illumination spectrum
- 50.: Illumination spectral bands
- 52.: Illumination filter system
- 54.: Illumination filter
- 56.: Input filter system
- 58.: Internal beam splitter
- 60.: Output spectrum
- 62.: Output spectral bands
- 64.: Output filter system
- 66.: Output filter
- 68.: Viewer filter system
- 70.: Polarization filter system of viewer
- 72.: Polarization filter of output color image projector
- 74.: Digital light processor
- 75.: Rotating filter wheel
- 76.: Patient
- 78.: Observer
- 80.: Screen
- 82.: Housing
- 84.: Stand
- 86.: Fibre optics
- f_{I}: Frame rate of camera system
- f_{L}: Frame rate of illumination color image projector
- f_{O}: Frame rate of output color image projector

## Claims

1. Observation apparatus (1) for visual enhancement of an observed object (2), comprising an output color image projector (8) for projecting a time sequence (31) of output color images (32) onto a projection area (40) on the object, the output color image projector (8) having an output spectrum (60) consisting of a set of output spectral bands (62) in the visible-light range, a multispectral camera system (4) for capturing a time sequence (21) of input color images (22) from the projection area (40) in at least two input spectral bands (42) in the visible-light range, the input spectral bands (42) being different from the output spectral bands (62), wherein the observation apparatus (1) is configured to compute the output color images (32) based on the input color images (22) and to project the output color images (32) onto the projection area (40) in real time, **characterised by** a visible-light transmitting viewer (10) for use by an observer (78) and for being directed onto the projection area (40) and comprising a viewer filter system (68) restricting light transmission to the output spectral bands (62), so that contrast is increased while at the same time allowing the observer (78) to view the object (2) and the output color images (32) at the output spectral bands (62), the viewer (10) using a separate imaging system operating on the same projection area (40) which is functionally separated from the multispectral camera system (4) and the output color image projector (8) by using different input (42) and output spectral bands (62).

2. Observation apparatus (1) according to claim 1, wherein the viewer (10) is an ocular or an observation screen.

3. Observation apparatus (1) according to claim 1 or 2, wherein an input spectrum (46) of the camera system (4) consists of more than four input spectral bands (42).

4. Observation apparatus (1) according to any one of clams 1 to 3, wherein the output color image projector (8) and the viewer (10) each comprise a polarization filter (70, 72).

5. Observation apparatus (1) according to any one of claims 1 to 4, wherein an input color image (22) of the time sequence (21) of input color images (22) and a corresponding output color image (32) of the time sequence (31) of output color images (32) are spatially congruent and have the same orientation.

6. Observation apparatus (1) according to any one of claims 1 to 5, wherein a common optical system comprising a common lens system (12) is provided for the output color image projector (8) and the camera system (4).

7. Observation apparatus (1) according to claim 6, wherein the camera system (4) and/or the output color image projector (8) are located remote from the object (2) and wherein the common optical system comprises fibre optics.

8. Method for visually enhancing the observation of an object (2), comprising the steps of capturing, by a multispectral system (4), a time sequence of input color images (22) in at least two input spectral bands (42) in the visible-light range from an observation area (40) on the object (2), computing in real time output color images (32) based on the input color images (22) and projecting, by an output color image projector (8), in real time the output color images (32) onto the observation area (40) using an output spectrum (60) of a set of output spectral bands (62) in the visible light range, wherein the output spectral bands (62) differ from the input spectral bands (42), further comprising the step of providing a visible-light transmitting viewer (10) for being directed onto and for observing the observation area (40) by an observer (78), the viewer (10) comprising a viewer filter system (68) restricting light transmission to the output spectral bands (62) so that contrast is increased, while at the same time allowing the observer (78) to view the object (2) and the output color images (32) at the output spectral bands (62).

9. Method according to claim 8, further comprising the step of illuminating the observation area (40) with an illumination spectrum (48) consisting of at least a subset of the input spectral bands (42).

## Patentansprüche

1. Beobachtungsvorrichtung (1) zur optischen Verbesserung eines beobachteten Objekts (2), umfassend einen Ausgangsfarbbild-Projektor (8) zum Projizieren einer Zeitsequenz (31) von Ausgangsfarbbildern (32) auf eine Projektionsfläche (40) am Objekt, wobei der Ausgangsfarbbild-Projektor (8) ein Ausgangsspektrum (60) bestehend aus einer Menge von Ausgangsspektralbändern (62) im sichtbaren Lichtbereich aufweist, ein Multispektral-Kamerasystem (4) zum Erfassen einer Zeitsequenz (21) von Ausgangsfarbbildern (22) von der Projektionsfläche (40) in wenigstens zwei Ausgangsspektralbändern (42) im sichtbaren Lichtbereich, wobei sich die Eingangsspektralbänder (42) von den Ausgangsspektralbändern (62) unterscheiden, wobei die Beobachtungsvorrichtung (1) zum Berechnen der Ausgangsfarbbilder (32) auf der Basis der Eingangsfarbbilder (22) und zum Projizieren der Ausgangsfarbbilder (32) auf die Projektionsfläche (40) in Echtzeit ausgebildet ist, **gekennzeichnet durch** einen sichtbares Licht übertragenden Betrachter (10) zur Verwendung durch einen Beobachter (78) und zum Ausrichten auf die Projektionsfläche (40) und umfassend ein die Lichtübertragung auf die Ausgangsspektralbänder (62) beschränkendes Betrachterfiltersystem (68), so dass der Kontrast erhöht wird, während gleichzeitig dem Beobachter (78) ermöglicht wird, das Objekt (2) und die Ausgangsfarbbilder (32) in den Ausgangsspektralbändern (62) zu betrachten, wobei der Betrachter (10) ein separates auf der gleichen Projektionsfläche (40) betriebenes Bildgebungssystem verwendet, das funktional vom Multispektral-Kamerasystem (4) und Ausgangsfarbbild-Projektor (8) durch Verwenden verschiedener Eingangsspektralbänder (42) und Ausgangsspektralbänder (62) getrennt ist.

2. Beobachtungsvorrichtung (1) nach Anspruch 1, wobei der Betrachter (10) ein Okular oder ein Beobachtungsbildschirm ist.

3. Beobachtungsvorrichtung (1) nach Anspruch 1 oder 2, wobei ein Eingangsspektrum (46) des Kamerasystems (4) aus mehr als vier Eingangsspektralbändern (42) besteht.

4. Beobachtungsvorrichtung (1) nach einem der Ansprüche 1 bis 3, wobei der Ausgangsfarbbild-Projektor (8) und der Betrachter (10) jeweils ein Polarisationsfilter (70, 72) umfassen.

5. Beobachtungsvorrichtung (1) nach einem der Ansprüche 1 bis 4, wobei ein Eingangsfarbbild (22) der Zeitsequenz (21) von Eingangsfarbbildern (22) und ein entsprechendes Ausgangsfarbbild (32) der Zeitsequenz (31) von Ausgangsfarbbildern (32) räumlich kongruent sind und die gleiche Ausrichtung aufweisen.

6. Beobachtungsvorrichtung (1) nach einem der Ansprüche 1 bis 5, wobei ein gemeinsames optisches System umfassend ein gemeinsames Linsensystem (12) für den Ausgangsfarbbild-Projektor (8) und das Kamerasystem (4) bereitgestellt wird.

7. Beobachtungsvorrichtung (1) nach Anspruch 6, wobei das Kamerasystem (4) und/oder der Ausgangsfarbbild-Projektor (8) entfernt vom Objekt (2) angeordnet sind und wobei das gemeinsame optische System Faseroptik umfasst.

8. Verfahren zum optischen Verbessern der Beobachtung eines Objekts (2), umfassend den Schritt zum Erfassen einer Zeitsequenz von Eingangsfarbbildern (22) durch ein Multispektralsystem (4) in wenigstens zwei Eingangsspektralbändern (42) im sichtbaren Lichtbereich von einer Beobachtungsfläche (40) am Objekt (2), Berechnen von Ausgangsfarbbildern (32) in Echtzeit auf der Basis der Eingangsfarbbilder (22) und Projizieren der Ausgangsfarbbilder (32) durch einen Ausgangsfarbbild-Projektor (8) in Echtzeit auf die Beobachtungsfläche (40) unter Verwendung eines Ausgangsspektrums (60) einer Menge von Ausgangsspektralbändern (62) im sichtbaren Lichtbereich, wobei sich die Ausgangsspektralbänder (62) von den Eingangsspektralbändern (42) unterscheiden, ferner umfassend den Schritt zum Bereitstellen eines sichtbares Licht übertragenden Betrachters (10) zum Ausrichten auf die Beobachtungsfläche (40) und Beobachten von dieser durch einen Beobachter (78), wobei der Betrachter (10) ein die Lichtübertragung auf die Ausgangsspektralbänder (62) beschränkendes Betrachterfiltersystem (68) umfasst, so dass der Kontrast erhöht wird, während gleichzeitig dem Beobachter (78) ermöglicht wird, das Objekt (2) und die Ausgangsfarbbilder (32) in den Ausgangsspektralbändern (62) zu betrachten.

9. Verfahren nach Anspruch 8, ferner umfassend den Schritt zum Beleuchten des Beobachtungsbereichs (40) mit einem Beleuchtungsspektrum (48) bestehend aus wenigstens einer Teilmenge der Eingangsspektralbänder (42).

## Revendications

1. Appareil d'observation (1) pour l'amélioration visuelle d'un objet (2) observé, comprenant un projecteur d'image de couleur de sortie (8) pour projeter une séquence de temps (31) d'images de couleur de sortie (32) sur une surface de projection (40) sur l'objet, le projecteur d'image de couleur de sortie (8) ayant un spectre de sortie (60) constitué d'un ensemble de bandes spectrales de sortie (62) dans la plage de la lumière visible, un système caméra (4) multispectral pour capturer une séquence de temps (21) d'images de couleur d'entrée (22) à partir de la surface de projection (40) dans au moins deux bandes spectrales d'entrée (42) dans la plage de la lumière visible, les bandes spectrales d'entrée (42) étant différentes des bandes spectrales de sortie (62), où l'appareil d'observation (1) est configuré pour calculer les images de couleur de sortie (32) sur la base des images de couleur d'entrée (22) et pour projeter les images de couleur de sortie (32) sur la surface de projection (40) en temps réel, **caractérisé par** un dispositif de visualisation de transmission de lumière visible (10) pour l'utilisation par un observateur (78) et pour être dirigé sur la surface de projection (40) et comprenant un système de filtrage de visualisation (68) restreignant la transmission de lumière vers les bandes spectrales de sortie (62), de sorte que le contraste est accru tout en permettant en même temps à l'observateur (78) de visualiser l'objet (2) et les images de couleur de sortie (32) aux bandes spectrales de sortie (62), le dispositif de visualisation (10) utilisant un système d'imagerie séparé fonctionnant sur la même surface de projection (40) qui est fonctionnellement séparé du système caméra (4) multispectral et le projecteur d'image de couleur de sortie (8) en utilisant des bandes spectrales d'entrée (42) et de sortie (62) différentes.

2. Appareil d'observation (1) selon la revendication 1, dans lequel le dispositif de visualisation (10) est un oculaire ou un écran d'observation.

3. Appareil d'observation (1) selon la revendication 1 ou 2, dans lequel un spectre d'entrée (46) du système caméra (4) est constitué de plus de quatre bandes spectrales d'entrée (42).

4. Appareil d'observation (1) selon l'une quelconque des revendications 1 à 3, dans lequel le projecteur d'image de couleur de sortie (8) et le dispositif de visualisation (10) comprennent chacun un filtre de polarisation (70, 72).

5. Appareil d'observation (1) selon l'une quelconque des revendications 1 à 4, dans lequel une image de couleur d'entrée (22) de la séquence de temps (21) des images de couleur d'entrée (22) et une image de couleur de sortie (32) correspondante de la séquence de temps (31) des images de couleur de sortie (32) sont spatialement congruentes et présentent la même orientation.

6. Appareil d'observation (1) selon l'une quelconque des revendications 1 à 5, dans lequel un système optique commun comprenant un système de lentille commun (12) est fourni pour le projecteur d'image de couleur de sortie (8) et le système caméra (4).

7. Appareil d'observation (1) selon la revendication 6, dans lequel le système caméra (4) et/ou le projecteur d'image de couleur de sortie (8) sont localisés loin de l'objet (2) et où le système optique commun comprend une fibre optique.

8. Procédé d'amélioration visuellement de l'observation d'un objet (2), comprenant les étapes de capture, par un système multispectral (4), d'une séquence de temps d'images de couleur d'entrée (22) dans au moins deux bandes spectrales d'entrée (42) dans la plage de la lumière visible à partir d'une surface d'observation (40) sur l'objet (2), le calcul en temps réel des images de couleur de sortie (32) sur la base des images de couleur d'entrée (22) et la projection, par un projecteur d'image de couleur de sortie (8), en temps réel des images de couleur de sortie (32) sur la surface d'observation (40) en utilisant un spectre de sortie (60) d'un ensemble de bandes spectrales de sortie (62) dans la plage de la lumière visible, où les bandes spectrales de sortie (62) diffèrent des bandes spectrales d'entrée (42), comprenant en outre l'étape de fourniture d'un dispositif de visualisation de transmission de lumière visible (10) pour être dirigée sur, et pour l'observation de, la surface d'observation (40) par un observateur (78), le dispositif de visualisation (10) comprenant un système de filtrage de visualisation (68) restreignant la transmission de lumière aux bandes spectrales de sortie (62) de sorte que le contraste est accru, tout en permettant en même temps à l'observateur (78) de visualiser l'objet (2) et les images de couleur de sortie (32) au niveau des bandes spectrales de sortie (62).

9. Procédé selon la revendication 8, comprenant en outre l'étape d'éclairage de la surface d'observation (40) avec un spectre d'éclairage (48) constitué d'au moins un sous-ensemble des bandes spectrales d'entrée (42).
